# EUROPEAN PATENT APPLICATION

(11) **EP 0 793 950 A1**
(43) Date of publication of application: **10.09.1997**
(21) Application number: 96920839.6
(22) Date of filing: 28.06.1996
(51) Int. Cl.: A61F 5/14, A43B 13/14

(54) **IMPROVEMENTS TO SHOE SOLES FOR RELIEVING THE PAIN IN THE BACK**

(30) Priority: 18.07.1995 ES 9501977 U
(71) Applicant: Oyanedel Neira, Jorge, 29738 Malaga (ES)
(72) Inventor: Oyanedel Neira, Jorge, 29738 Malaga (ES)
(86) International application number: ES9600140
(87) International publication number: WO9703628

(57) **Abstract**

Improvements to shoe soles intended to relieve the pain in the back, the soles being specially designed for the housing of feet insoles, held by any known method, and made, although not restricted to it, of natural material , such as polyurethane or the like, being the type that presents a different level between the rear part (5) and the fore part (4) the rear part being lower The disclosed soles incorporate a wall (2) which surrounds the external edge of the platform and which is raised (3) progressively as it approaches the rear part, without being restricted to a predetermined height at said wall.

## Description

### PURPOSE OF THE INVENTION

The invention as expressed in the title of this Descriptive Memory, refers to improvements to shoe soles , being their aim, to relieve back pain provoked by impingement arising from excessive closeness of two adjacent vertebrae and make its use available to persons with abnormal plantar angle (plane feet) or tender feet..

The principle by which the mentioned effect is produced, is the capacity of the Human Nervous System to enact the so called "Posture Reflex". This is a Reflex Act by which physical posture is maintained or recovered and depends on Central Nervous System autonomously regulated systems. The body has the ability to recover the erected position by itself without the action of volition, straightening the vertebral lordosis (curve of the back) by means of shifting posture, moving the body in the anterior direction. If the horizontal plane is varied when a subject is in a standing position (upright position), elevating the anterior end, such individual with tend to keep balance, this is the upright position, by a reflex act. The reflex mechanism that the body uses to keep the head and center of gravity into balance exercises traction over the vertebral column , varies the pelvic angle with respect to the horizontal plane and modifies the angle between adjacent vertebrae surfaces tending to separate them in accordance with the variation of the angle with the horizontal antero-posterior plane. To illustrate this point, we can imagine that the individual will be in the upright position in a flat surface that begins to be elevated by its front end. As the plane is being raised, the subject tends to lean forwards in order to keep parallel to the Normal (vertical position). A rectangle triangle is then described where the individual looks to the opposed catete, standing on the hypotenuse. The Central Nervous System and body structures by themselves take care of increasing the radius of the curve described by the lordosis (physiological curves of the spine). This fact has a direct influence over pressure exercised on intermediate structures, since by increasing the radius of the lordosis, said structures become separated, thus preventing the cause of pain. At the same time the variation of the angle of the pelvis towards the horizontal plane, tends to prevent the colloidal contents of the abdominal cavity to be poured towards the front, so preventing said contents to pull from the posterior abdominal wall where the spine bone and muscle bundle is adhered. That is how this pulling-forward tension exercised by the abdominal organs over the lumbar lordosis is relieved.

Indeed, outsoles manufactured with the improvements proposed by the invention which will be detailed later, induce an artificial angulation because of its special design, that can be attached to a normal insole for feet, making the body to produce this adjustment which happily modifies the intervertebral space increasing its clearance. The problem of back pain is solved artificially separating the oppressing vertebrae by means of the proposed invention. At the same time the flat platform of the proposed invention makes it usable by persons with flat or tender feet, thus avoiding the hardness of soles or models with prominences. The Brain Mechanism which drives the Posture Keeping is called Posture Reflex and its known mediators in man are two systems : The Golgi Tendinous Organ and The Gamma Efferent System. They can be found in muscles and joints and respond with nervous impulses to shortening or stretching of muscle fibers. Any movement, change in position is recorded by these proprioceptors and reported to Brain Centers which device tension or relaxation of muscles or bundles of muscles in order to recover postural balance.

A change in posture is produced then, artificially by knowing how the principle works and directing it to that certain group of muscles that are related with traction of involved vertebrae by action of paravertebral musculature and leveling the pelvic line towards the horizontal plane by action of the ischio-tibialis bundles of muscles. Vertebral body separation and relief of pressure over the annulus are an immediate consequence of this fact.

### BACKGROUND OF THE INVENTION

There is a background information about devices with the effects that the invention proposes for the relief of back pain. On this line we can mention soles with prominences to adapt to the fingers of a normal foot, with an elevation for the plantar angle and a groove for the support of the external border of the foot, a groove and a hole for the support of the heel in a way that fore and rear planes of the soles are found at different levels, the rear being inferior. This system has a drawback in the sense that these soles are of deficient cosmetic appearance and cause damages to people with tender feet and cannot be used by people of flat feet since they inflict unbearable pain. At the same time said soles are difficult to maneuver by the shoe mounting machines in the fabrication process, since they are bulky and thick in the forefoot aspect and interfere in the passage of the blade that mounts the vamp (tarsal cover of the shoe), destroying the sole in most of the cases, which makes the manufacturing process to be laborious and expensive.

Shoes with partial elevations are known to be used for the correction of malformations or defects of the feet themselves but in all cases the anterior aspect is lower than the rear, not existing any surrounding wall which is higher in the rear half improving the aesthetic aspect, neither showing a difference in antero-posterior height nor the fact of being an outsole instead of an insole.

Although there are current ways of relieving back pain, they do not disclose the features of the invention and are additionally iatrogenic, inducing adverse side effects with the long term use.

The invention may be used with no limitations of time and with no risk of adverse side effects.

### DESCRIPTION OF THE INVENTION

The advantages presented by the outsoles for shoes, proposed by the invention are the following :

It is a non toxic, non iatrogenic product, adverse side effects have not been evidenced, it does not wear out, since the patient may use them for as long as wanted during life.

In addition, it proposes a clear advantage over other conservative procedures to relieve pain arising from vertebral closeness, by being completely innocuous and because of its design, it may be used by a higher amount of population.

It serves as an outsole for any kind of insole for shoes, whether orthopaedical or not. It is made of a flexible substance that may be polyurethane and presents the following basic features :
- A platform with a flat surface where an insole for feet can be placed.
- A wall that surrounds the surface of the platform that increases its height as long as it goes in the backwards direction with the purpose of improving the aesthetical appearance before its fore portion being higher than the rear portion.
- A different level of height between the anterior and posterior portion of the platform, as a slope that goes down as it runs down from anterior to posterior. This difference will make tension to appear in the muscles of the lumbar portion and will tense the ischio-tibialis and semimembranaceus muscles, which will make the pelvis to move towards the horizontal plane. The Posture Reflex will lead a readjustment of the body's posture to keep balance and by this act, vertebral separation, specially vertebral bodies and processes of the lumbar vertebrae. Pressure is then released from the intervertebral disc and intermediate and surrounding structures, preventing the possibility of inflammatory reactions due to contact with hard structures. When inflammation ceases, volume decreases and pressure over nervous elements is then ceased, eliminating pain. Horizontalization of the pelvic line relieves the tension that viscerae exercise on the posterior wall of the abdomen, lowering the tendency to empty the cavity induced by gravity, thus decreasing the force that tends to increase closeness of adjacent vertebrae in the lumbar spine.

The outsoles proposed by the invention act as inductors of posture balance which prevents pain provoked by vertebral impingement, increases the plantar angle, increases the radius of the circle described by physiological lordosis and relieves pressure over the intervertebral disc

The tendency of the pelvis to move to the horizontal plane by the action of the muscles inserted from the ischion to the lower limb, triggered by Posture Reflex release the tension produced by the force of gravity over the anterior aspect of the back since it tends to contain and not to empty the contents of the cavity.

With these outsoles a separation of the processus spinales of adjacent vertebrae is produced and the pain called sciatica is eliminated.

In order to facilitate the understanding of the features of the invention and as part of this Descriptive Memory, a sheet of figures is included whose drawings for purposes of illustration and not for limitation have been depicted as follows :

### BRIEF DESCRIPTION OF ATTACHED DRAWINGS

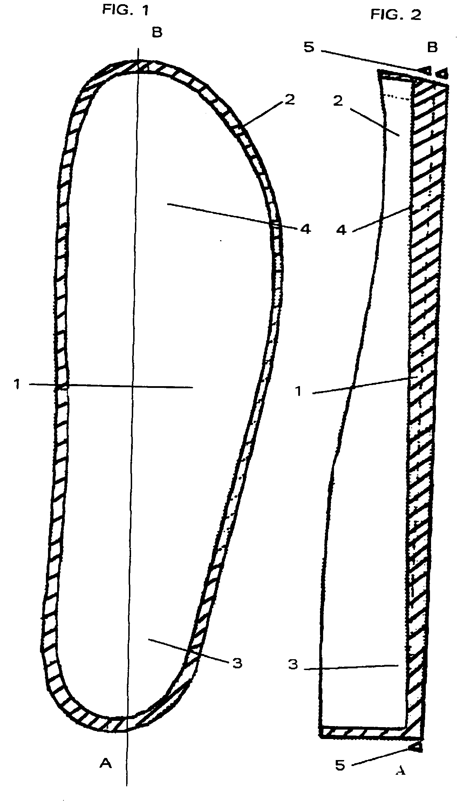

Figure 1.- A view from above showing the improvements proposed by the invention.

Figure 2.- A section of a full length through a cut line A-B in figure 1.

### DESCRIPTION OF THE PREFERRED FORM OF REALIZATION

With reference to the numbers in the above drawings, we can see that with the improvements introduced in outsoles for shoes proposed by the invention, a new concept of outsole for shoes is achieved, presenting features very different from those known so far, based on the peculiar structure presented by the upper aspect them.

Firstly, we can see that the outsole referred to as number 1 and corresponding to the right foot of the user, conforms a flat surface, surrounded by a wall (2) which shape resembles a fence surrounding the whole of the platform, where we see an increased height on both sides as it comes closer to the rear part (3). In the rear aspect of the platform the beginning of a slope is located in the place corresponding to where the heel is situated in any other sole being this in a lower level (5) than the anterior aspect (4), the opposite that we see in a common shoe.

## Claims

1. Improvements to shoe soles intended to relieve the pain in the back, the soles being specially designed for the housing of feet insoles, held by any known method, and made, although not restricted to it, of natural material , such as polyurethane or the like, being the type that presents a different level between the rear part (5) and the fore part (4) the rear part being lower. The disclosed soles incorporate a wall (2) which surrounds the external edge of the platform and which is raised (3) progressively as it approaches the rear part, without being restricted to a predetermined height at said wall.
